(19) Europäisches Patentamt

**European Patent Office**

**Office européen des brevets**

(11) Numéro de publication: **0 305 288 B1**

(12) **FASCICULE DE BREVET EUROPEEN**

(45) Date de publication de fascicule du brevet: **15.04.92** (51) Int. Cl.⁵: **C07C 17/38**, C07C 17/10

(21) Numéro de dépôt: **88402146.0**

(22) Date de dépôt: **23.08.88**

(54) **Procédé d'élimination du chlore.**

(30) Priorité: **27.08.87 FR 8711990**

(43) Date de publication de la demande:
**01.03.89 Bulletin 89/09**

(45) Mention de la délivrance du brevet:
**15.04.92 Bulletin 92/16**

(84) Etats contractants désignés:
**AT BE CH DE ES FR GB GR IT LI LU NL SE**

(56) Documents cités:
**FR-A- 890 730**
**FR-A- 1 181 751**
**FR-A- 1 362 507**

(73) Titulaire: **ATOCHEM**
**4 & 8, Cours Michelet La Défense 10**
**F-92800 Puteaux(FR)**

(72) Inventeur: **Masini, Jean-Jacques**
**35 Rue Jean Penet**
**F-69630 Chaponost(FR)**
Inventeur: **Collier, Bertrand**
**7 Avenue de Gadagne**
**F-69230 Saint-Genis Laval(FR)**

(74) Mandataire: **Leboulenger, Jean et al**
**ATOCHEM Département Propriété Industriel-**
**le**
**F-92091 Paris la Défense 10 Cédex 42(FR)**

# Description

La présente invention concerne un procédé pour éliminer le chlore contenu dans un mélange. Elle concerne plus particulièrement un mélange contenant au moins un produit organique non saturé en chlore substitué. Dans l'industrie chimique on trouve souvent des mélanges de produits contenant du chlore libre dissous et des produits organiques non saturés en chlore, par exemple lors de la synthèse des trichloroethanes. Le brevet US 3 474 018 décrit un procédé de synthèse du 1,1,1-trichloroethane ; l'une des étapes du procédé est une séparation de chlorure d'éthyle d'un mélange de chloroethanes contenant du chlore dissous. La distillation de tels mélanges contenant du chlore peut entraîner des corrosions (voir KIRK OTHMER, 3ème édition, Vol. 1, pages 835 et suivantes). Le brevet US 3 344 197 mentionne aussi les risques de corrosion causés par la présence du chlore.

On a maintenant trouvé un nouveau procédé qui permet d'éliminer toute trace de chlore sans avoir à utiliser des distillations d'autant plus difficiles qu'il s'agit de traces.

La présente invention concerne un procédé pour éliminer le chlore contenu dans un mélange comprenant au moins un produit organique non saturé en chlore substitué caractérisé en ce que ledit mélange est soumis à une chloration radicalaire.

L'invention s'intéresse à tous les mélanges mais plus particulièrement à ceux contenant essentiellement des produits organiques.

L'invention s'applique aussi à un mélange ne contenant que du chlore libre et un produit organique non saturé en chlore substitué, ce mélange contenant éventuellement un solvant.

Le produit organique non saturé en chlore substitué est un produit ayant encore au moins un atome d'hydrogène pouvant être substitué par le chlore.

L'invention peut s'appliquer à des mélanges contenant toute proportion de chlore, mais elle est surtout utile pour les mélanges contenant peu de chlore. Quand le mélange contient beaucoup de chlore, c'est-à-dire plus de 5 ou 10 % en poids, on peut traiter le mélange par des flash et/ou des évaporations/condensations fractionnées, ou des distillations partielles pour éliminer une grande partie du chlore. On applique l'invention de préférence aux mélanges contenant (en poids) moins de 5 % et le plus souvent moins de 0,1 % de chlore.

Le mélange doit contenir un ou plusieurs produits organiques non saturés en chlore substitué en quantité suffisante pour consommer le chlore libre. La chloration radicalaire est une réaction connue en soi.

Ce procédé peut être mis en oeuvre avec une initiation chimique ou une initiation photochimique. Dans le cas d'une initiation chimique, on pourra utiliser les initiateurs connus pour les réactions de chloration.

A titre purement indicatif, on mentionnera les composés diazoïques tels que le 2,2'-azo-bis-isobutyronitrile ou le 2,2'-azo-bis 2,3 diméthylvaléronitrile ou encore les composés péroxydiques tels que le péroxyde de lauroyle, le péroxyde de benzoyle. D'une manière générale, les composés peuvent être mis en oeuvre tels quels ou sous forme de solution, notamment dans un chloroalcane.

Ou peut, dans le procédé conforme à l'invention, obtenir un taux de transformation de chlore supérieur à 99 % et plus précisément supérieur à 99,5 % avec une quantité d'initiateur généralement comprise entre $10^{-2}$ et $2.10^{-6}$ et de préférence entre $5.10^{-3}$ et $10^{-5}$ mole d'initiateur par mole de chlore, pour les initiateurs ayant une constante de dissociation dans le toluène comprise entre $5.10^{-7}$ et $5.10^{-4}$ $sec^{-1}$ et de préférence entre $10^{-6}$ et $2.10^{-4}$ $sec^{-1}$.

A titre d'information, et dans l'hypothèse d'un initiateur photochimique, on peut obtenir un taux de transformation en chlore supérieur à 99 % et plus précisément supérieur à 99,5 %, pour une luminance comprise entre 0,5 et 2 $W/cm^2$ (puissance électrique totale de l'émetteur rapportée à la surface externe de l'enveloppe correspondant à la longueur de l'arc électrique du luminant) et une consommation électrique correspondant à l'émission photoélectrique comprise entre 0,1 et 100 Wh/mole et plus particulièrement entre 0,5 et 70 Wh/mole de chlore mis en oeuvre.

On peut utiliser une capacité quelconque pour faire la réaction : un réservoir, une tuyauterie, un plateau d'une colonne à distiller, le rebouilleur d'une colonne... Il suffit seulement de bien distribuer l'initiateur ou les radiations dans tout le mélange et de respecter un temps de séjour pour consommer le chlore dans les proportions indiquées ci-dessus.

On peut opérer en phase gazeuse, liquide ou avec un mélange partiellement liquide. Bien qu'on puisse opérer dans une large plage de température, on préfère se placer au dessus de 20°C et de préférence entre 40 et 100°C. La cinétique de disparition du chlore augmente avec la température. Le temps de séjour nécessaire pour consommer tout le chlore est généralement compris entre quelques secondes et une heure pour des températures supérieures à 50°C. Bien qu'on puisse opérer dans une large plage de pression, on opère généralement entre 1 et 50 bars et de préférence entre 7 et 13 bars.

L'invention est particulièrement utile pour les mélanges de chloroalcanes. Dans la synthèse des chloroalcanes on fait réagir du chlore sur des alcanes ou des chloroalcanes ayant encore des sites substituables par le chlore. Les alcanes et les chloralcanes peuvent être en phase gazeuse ou liquide avec ou sans solvant. A la fin de la réaction on obtient des alcanes et/ou des chloroalcanes plus ou moins substitués, de l'acide chlorhydrique, du chlore n'ayant pas réagi et éventuellement le solvant. Il est nécessaire de séparer les chloroalcanes soit pour les obtenir séparément et purs, soit pour les recycler dans une autre partie du procédé. De tels procédés sont décrits par exemple dans la demande de brevet européen EP 0 128 818 et dans la demande britannique GB 2 158 067.

Les produits, liquides, gazeux, ou liquides et gazeux quittant le(s) réacteur(s) c'est-à-dire un mélange de chloroalcanes, d'HCl et de chlore, sont traités par un ensemble de colonnes à distiller qui permet de séparer successivement l'HCl des chloroalcanes puis chacun des chloroalcanes. Le chlore se concentre dans la section d'épuisement de la colonne de séparation d'HCl des chloroalcanes.

On applique l'invention au mélange présent dans la section d'épuisement de cette colonne. On peut injecter l'initiateur chimique dans la colonne ou disposer des émetteurs UV dans la colonne.

Avantageusement (i) on soutire le mélange liquide pris sur un plateau de la colonne ou dans le cas d'une colonne à garnissage sur un plateau de distribution, (ii) on fait une chloration radicalaire de ce mélange, puis (iii) on réinjecte le mélange épuré en chlore dans la colonne.

Une mise en oeuvre particulière du procédé de l'invention est illustrée à l'aide du schéma sur la planche jointe.

Une colonne à distiller (10) équipée d'un rebouilleur (11) d'un condenseur de reflux (12) est alimentée en (1) avec un mélange de chloroalcanes, d'HCl et de chlore. En (2) on soutire l'HCl et en (3) les chloroalcanes.

Le réacteur de chloration (13) est alimenté en liquide à partir d'un plateau n + 1 par un conduit (4) puis la phase liquide pouvant contenir de l'HCl dissous quitte le réacteur (13) et retourne dans la colonne par le conduit (5) sur le plateau n. Un conduit (6) relie la phase vapeur du réacteur (13) et la phase vapeur de la colonne (10). Le réacteur (13) contient un ou plusieurs émetteurs U.V. (14). Quand l'émetteur U.V. fonctionne, les deux courants (2) et (3) ne contiennent pas de chlore, le chlore contenu dans le courant (1) a donc été consommé ; quand l'émetteur U.V. ne fonctionne pas le chlore contenu dans le courant (1) passe dans le courant (3) de chloroalcanes.

La présente invention concerne aussi un procédé de synthèse des chloroalcanes utilisant 2 réacteurs en série caractérisé en ce que :

a) dans le premier réacteur on synthètise des chloroalcanes par chloration radicalaire,

b) on traite les produits obtenus pour concentrer le chlore qui n'a pas réagi,

c) on soumet à une chloration radicalaire le mélange résultant qui contient du chlore et au moins un chloroalcane qui peut réagir du chlore sous conditions de chloration radicalaire pour éliminer le chlore contenu dans ledit mélange, la réaction de l'étape c ayant lieu dans la section d'épuisement d'une colonne de séparation d'HCl d'un mélange HCl, chlore, chloralcanes.

Le premier des réacteurs en série n'est pas forcément un seul réacteur mais doit être entendu comme un ensemble réactionnel, c'est par exemple un jeu de 2 réacteurs en parallèle comme décrits dans la demande de brevet européen 128 818 ou un réacteur boucle comme dans le brevet britannique GB 2 158 067. Dans ce premier réacteur on introduit des réactifs et du chlore. On obtient un mélange contenant HCl, des chloroalcanes et du chlore qui n'a pas réagit. On peut séparer par distillation les produits ; l'HCl étant le plus volatil est séparé le premier, puis on sépare les chloroalcanes en commençant par les plus volatils et qui sont aussi les moins saturés en chlore, puis en fin de distillation on sort les chloroalcanes saturés. Le chlore accompagne les chloroalcanes non saturés. Par effet de distillation, le chlore contenu dans les produits venant de l'étape a) est concentré. Selon les puretés recherchées pour les produits, on est amené à distiller des produits contenant du chlore libre. Pour éviter des séparations diffi ciles pouvant provoquer des corrosions, la demanderesse a eu l'idée d'éliminer le chlore contenu dans ces mélanges contenant un chloroalcane non saturé en chlore substitué.

Dans une mise en oeuvre préférée de l'invention, le mélange d'HCl de chlore et de chloroalcanes sortant de l'étape a) éventuellement partiellement gazeux est introduit dans une colonne à distiller conventionnelle (étape b). En tête, on sort l'HCl, dans la section d'épuisement, de préférence à hauteur d'un plateau théorique dont la température est comprise entre 40 et 100°C, on réalise l'étape c) et en pied sortent les chloroalcanes.

L'avantage d'un tel procédé est que dans l'étape a) on peut faire une chloration radicalaire sans avoir à surveiller de façon précise l'excès éventuel de chlore. Un autre avantage est qu'on élimine la présence de chlore dans la partie inférieure de la colonne de séparation où la température est maximale, ce qui limite les problèmes éventuels de corrosion. Un autre avantage est qu'on limite la teneur en chlore dans le chloroalcane le plus léger, qui est obtenu en tête de la colonne suivante. Un autre avantage est que l'HCl sous produit est immédiatement séparé des produits de la réaction ; cet HCl part en tête de la colonne et on obtient en pied des chloroalcanes exempts de chlore.

## EXEMPLE

On utilise le dispositif représenté sur la planche jointe. La colonne comporte 50 plateaux, le réacteur (13) est disposé entre les plateaux 18 et 19 (numérotés en partant du bas de la colonne) et a la forme d'un tube vertical de diamètre 0,2 m et de hauteur 3,8 m dans lequel on dispose un émetteur U.V. d'une puissance de 10 KW.

La pression de la colonne est 12 bars effectifs, la température de tête - 25°C et la température de pied 114°C.

Le courant d'alimentation (1) a la composition suivante :
HCl 28 %
$CH_3Cl$ 8,5 %
$CH_2Cl_2$ 16,1 %
$CHCl_3$ 26,4 %
$CCl_4$ 21 %
$Cl_2$ 400 ppm

Les pourcentages sont en poids, le débit est 28250 kg/h et l'alimentation est sur le plateau 35. On constate que les courants (2) et (3) ne contiennent plus de chlore.

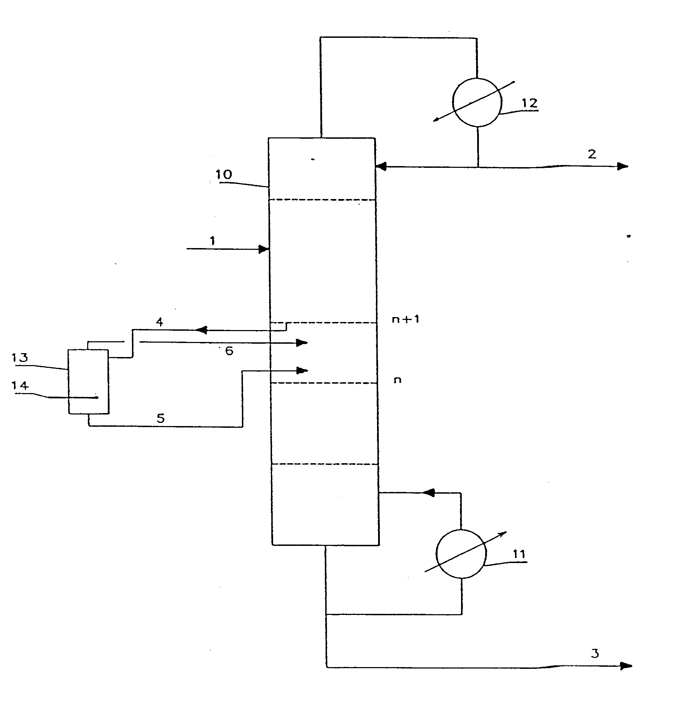

## Revendications

1. Procédé pour éliminer le chlore contenu dans un mélange comprenant au moins un chloroalcane pouvant réagir avec du chlore sous conditions de chloration radicalaire, ledit mélange étant présent dans la section d'épuisement d'une colonne de séparation d'HCl de chlore et de chloroalcanes caractérisé en ce qu'on soumet ledit mélange aux conditions d'une chloration radicalaire.

2. Procédé selon la revendication 1 caractérisé en ce eue le mélange contient en poids moins de 5 % de chlore et de préférence moins de 0,1 %.

3. Procédé de synthèse des chloroalcanes utilisant 2 réacteurs en série caractérisé en ce que :
   a) dans le premier réacteur on synthétise des chloroalcanes par chloration radicalaire,
   b) on traite les produits obtenus pour concentrer le chlore qui n'a pas réagi,
   c) on soumet à une chloration radicalaire le mélange résultant qui contient du chlore et au moins un chloroalcane qui peut réagir avec du chlore sous conditions de chloration radicalaire pour éliminer le chlore contenu dans ledit mélange, la réaction de l'étape c ayant lieu dans la section d'épuisement d'une colonne de séparation d'HCl d'un mélange HCl, chlore, chloroalcanes.

## Claims

1. Process for removing the chlorine present in a mixture containing at least one chloroalkane capable of reacting with chlorine under radical chlorination conditions, the said mixture being present in the stripping section of a column for separating HCl from chlorine and chloroalkanes, characterised in that the said mixture is subjected to the conditions of a radical chlorination.

2. Process according to Claim 1, characterised in that the mixture contains less than 5%, and preferably less than 0.1%, by weight of chlorine.

3. Process for the synthesis of chloroalkanes employing 2 reactors in series, characterised in that:
   a) chloroalkanes are synthesised by radical chlorination in the first reactor,
   b) the products obtained are treated to concentrate the unreacted chlorine, and
   c) the resultant mixture, which contains chlorine and at least one chloroalkane capable of reacting with chlorine under radical chlorination conditions, is subjected to a radical chlorination in order to remove the chlorine present in the said mixture, the reaction in stage c taking place in the stripping section of a column for separating HCl from a mixture of HCl, chlorine and chloroalkanes.

## Patentansprüche

1. Verfahren zur Entfernung von Chlor, enthalten in einer Mischung, die wenigstens ein Chloralkan enthält, welches mit Chlor unter den Bedingungen einer radikalischen Chlorierung reagieren kann, wobei diese Mischung in dem Abziehbereich einer Säule zur Trennung von HCl von Chlor und von Chloralkanen vorliegt, dadurch gekennzeichnet, daß man diese Mischung den Bedingungen einer radikalischen Chlorierung unterwirft.

2. Verfahren nach Anspruch 1, dadurch gekennzeichnet, daß die Mischung weniger als 5 Gew.% Chlor und vorzugsweise weniger als 0,1 Gew.% enthält.

3. Verfahren zur Synthese von Chloralkanen unter Verwendung von zwei hintereinandergeschalteten Reaktoren, dadurch gekennzeichnet, daß man
   a) in dem ersten Reaktor Chloralkane durch radikalische Chlorierung synthetisiert,

b) die erhaltenen Produkte zur Konzentrierung des nicht umgesetzten Chlors behandelt,

c) die erhaltene Mischung, die Chlor und wenigstens ein Chloralkan enthält, welches mit dem Chlor unter den Bedingungen einer radikalischen Chlorierung reagieren kann, zur Entfernung des Chlors in dieser Mischung einer radikalischen Chlorierung unterwirft, wobei die Reaktion der Stufe c in dem Abziehbereich einer Säule zur Trennung von HCl von einer Mischung aus HCl, Chlor und Chloralkanen stattfindet.